# EUROPÄISCHE PATENTANMELDUNG

(11) **EP 1 013 645 A1**
(43) Veröffentlichungstag der Anmeldung: **28.06.2000**
(21) Anmeldenummer: 99123983.1
(22) Anmeldetag: 07.12.1999
(51) Int. Cl.: C07D 207/32

(54) **Verfahren zum Herstellen von 2,4-Dimethyl-pyrrol**

(30) Priorität: 17.12.1998 DE 19858360
(71) Anmelder: BASF AKTIENGESELLSCHAFT, 67056 Ludwigshafen (DE)
(72) Erfinder: Gröning, Carsten, Dr., 68259 Mannheim (DE); Kemper, Reinhard, Dr., 69123 Heidelberg (DE); Frede, Markus, Dr., 69214 Eppelheim (DE); Ebel, Klaus, Dr., 68623 Lampertheim (DE)
(74) Vertreter: Isenbruck, Günter, Dr.

(57) **Zusammenfassung**

Es wird ein Verfahren zum Herstellen von 2,4-Dimethyl-pyrrol beschrieben, bei dem ein 2,4-Dimethyl-3,5-bis-alkoxycarbonyl-pyrrol mit 10 bis 30 %iger wäßriger Alkalilauge so lange unter Rückfluß erhitzt wird, bis der Feststoff in Lösung gegangen ist, das Reaktionsgemisch mit Säure neutralisiert und weiter am Rückfluß bis zur vollständigen Decarboxylierung erhitzt wird. Das Verfahren erlaubt es, unter wesentlich milderen, weniger aggressiven Bedingungen zu arbeiten, als es nach dem Stand der Technik bekannt ist.

## Beschreibung

Die Erfindung betrifft ein Verfahren zum Herstellen von 2,4-Dimethyl-pyrrol aus 2,4-Dimethyl-3,5-bis-alkoxycarbonyl-pyrrol durch Verseifen und Decarboxylieren.

Die Synthese des 2,4-Dimethyl-3,5-bis-alkoxycarbonyl-pyrrols ist zum Beispiel in Org. Syntheses, Coll. Vol. II, 202 - 204 (1943) beschrieben. Sie erfolgt vorzugsweise nach dem in der gleichzeitig eingereichten Patentanmeldung 198 58 352.4 (NAE 19980438) beschriebenen Verfahren durch Nitrosieren eines Acetessigsäurealkylesters. Hydrieren der 2-Nitrosoverbindung zum Amin mit Wasserstoff in Gegenwart eines Edelmetallkatalysators und Kondensieren der Aminoverbindung mit unsubstituiertem Acetessigester zum Pyrrolderivat.

Die Hydrolyse von 2,4-Dimethyl-3,5-bis-ethoxycarbonyl-pyrrol und die nachfolgende Decarboxylierung der Dicarbonsäure wird in Org. Synth., Coll. Vol. II, Seite 217 - 218 (1943) mit einer Ausbeute von 57 bis 63 % beschrieben. Dort wird der Ester mit konzentrierter (64 %) Kalilauge innerhalb von 2 bis 3 Stunden bei 130°C verseift. Die Decarboxylierung erfolgt dann durch Erhitzen des Reaktionsgemischs auf 160 bis 200°C. Der Nachteil dieses Verfahrens besteht darin, daß bei den benötigten hohen Temperaturen nur teure Nickelstähle gegenüber der Lauge beständig sind.

Höhere Ausbeuten (etwa 95 %) werden von Corvin et al in J. Am. Chem. Soc. 63, 1829 - 1834 (1941) angegeben, wonach hochkonzentrierte Kaliumhydroxidlösungen oder -schmelzen 4 bis 5 Stunden bei 160°C unter Druck mit dem Dicarbonsäurediethylester umgesetzt werden. Auch hier werden sehr aggressive Reaktionsgemische benötigt.

Aufgabe der Erfindung war es, ein Verfahren zum Herstellen von 2,4-Dimethyl-pyrrol aus 2,4-Dimethyl-3,5-bis-alkoxycarbonyl-pyrrol vorzuschlagen, das das Produkt in guter Ausbeute liefert und unter milderen Bedingungen durchgeführt werden kann, als es im Stand der Technik beschrieben ist.

Die Erfindung geht aus von einem Verfahren zum Herstellen von 2,4-Dimethyl-pyrrol, bei dem ein 2,4-Dimethyl-3,5-bis-alkoxycarbonyl-pyrrol mit starkem Alkali zur Dicarbonsäure verseift und die Dicarbonsäure thermisch decarboxyliert wird.

Das erfindungsgemäße Verfahren ist dadurch gekennzeichnet, daß man den Dicarbonsäurealkylester mit 10 bis 30 %iger wäßriger Alkali-, bevorzugt Natronlauge so lange unter Rückfluß erhitzt, bis der Feststoff in Lösung gegangen ist, das Reaktionsgemisch mit Säure neutralisiert und weiter am Rückfluß bis zur vollständigen Decarboxylierung das heißt, bis zum Ende der Gasentwicklung erhitzt.

In der ersten Stufe erfolgt die Verseifung der Estergruppen bevorzugt bei Normaldruck und mit einem stöchiometrischen Überschuß an Alkali. Die Rückflußtemperatur beginnt dann zunächst oberhalb der Siedetemperatur des entsprechenden Alkohols und steigt mit dessen Entfernung aus dem Gleichgewicht je nach Konzentration der Alkalilauge auf Werte oberhalb 100°C. Im allgemeinen erfolgt die Verseifung innerhalb von 5 bis 15 Stunden. Auch die Decarboxylierung - zumindest der ersten Carboxylgruppe - beginnt bereits unter diesen Bedingungen. So setzt häufig schon ab 90°C oder wenig darüber eine heftige Gasentwicklung ein. Das entstandene Alkanol kann vorteilhaft unter den Verfahrensbedingungen abdestilliert werden. Wenn die Reaktion abgeklungen und die eingesetzte Substanz gelöst ist, wird die Lösung - zweckmäßig nach kurzem Abkühlen zur Vermeidung einer heftigen Wärmeentwicklung - mit Säure, vorteilhaft einer nicht flüchtigen starken Mineralsäure, zum Beispiel Schwefelsäure oder Phosphorsäure, neutralisiert. Danach wird weiter am Rückfluß erhitzt, wobei das 2,4-Dimethyl-pyrrol zusammen mit Wasser übergeht und davon abgeschieden werden kann. Diese zweite Decarboxylierungsstufe kann auch unter Druck, zum Beispiel unter dem Eigendruck des Gemischs von etwa 10 bar bei Temperaturen im Bereich von etwa 130 bis 145°C durchgeführt werden. Das Arbeiten unter Normaldruck und mit Abdestillieren der Reaktionsprodukte wird im allgemeinen bevorzugt.

Der als Ausgangsstoff eingesetzte Dialkylester kann Alkylgruppen mit 1 bis 4 Kohlenstoffatomen haben. Dabei werden Methyl- und Ethylester bevorzugt, da sie besonders reaktionsfähig sind und die entstandenen Alkohole sich leichter abtrennen lassen.

Wenn der Dialkylester nach dem Verfahren der oben erwähnten Parallelanmeldung durch Nitrosieren, Hydrieren und Kondensieren aus dem Acetessigsäurealkylester hergestellt wird, kann die Verseifung und Decarboxylierung gemäß vorliegender Erfindung auch mit dem Rohprodukt durchgeführt werden, das nach katalytischer Hydrierung zum Amin und Kondensation zum Dimethyl-bis-alkoxycarbonyl-pyrrol erhalten wird und noch den Katalysator enthält. Dieser kann nach der Stufe der Esterverseifung besonders einfach durch Filtrieren abgetrennt werden.

Das erfindungsgemäße Verfahren liefert das gewünschte 2,4-Dimethyl-pyrrol in hoher Ausbeute, ohne daß besonders alkaliresistente Reaktionsgefäße und -geräte eingesetzt werden müssen. Die Nebenprodukte lassen sich teils in die Reaktionszone zurückführen, teils können sie in einfacher, umweltfreundlicher Weise entsorgt werden.

Die folgenden Beispiele erläutern einzelne vorteilhafte Ausführungsformen des erfindungsgemäßen Verfahrens. Mengenverhältnisse und Prozente sind dabei in Gewichtseinheiten zu verstehen, wenn nichts anderes angegeben ist.

### Beispiel 1

399 g (1,89 mol) 2,4-Dimethyl-3,5-bis-methoxycarbonyl-pyrrol, die durch Hydrieren von 2-Nitroso-acetessigsäuremethylester zur 2-Aminoverbindung und Kondensieren mit Acetessigsäuremethylester erhalten worden waren und noch etwa 4 g Katalysatorsubstanz auf Basis Palladium/Aktivkohle enthielten, wurden in 1250 g 20 %ige Natronlauge (6,25 mol NaOH) eingerührt und 10 Stunden unter Rückfluß erhitzt. Der organische Feststoff geht dabei in Lösung. Danach wurde der Katalysatorrückstand abfiltriert, das dunkle Filtrat wurde mit 284 g 95 %iger Schwefelsäure auf pH 7 gebracht und dann 10 Stunden auf 135 bis 140°C unter dem sich einstellenden Eigendruck der Mischung (9 bis 10 bar) 18 Stunden erhitzt.

Nach dem Abkühlen lag ein dreiphasiges Gemisch vor: eine flüssige obere organische, produkthaltige Phase, eine untere wäßrige Phase und ein Feststoff als Bodenkörper. Der Feststoff (207 g) bestand hauptsächlich aus Natriumsulfat und Natriumcarbonat und wurde abfiltriert.

Die organische Phase wurde abgetrennt und die wäßrige Phase mit dem gleichen Volumen Ethylacetat extrahiert. Die organischen Phasen wurden vereinigt, über Natriumsulfat getrocknet und das Ethylacetat abdestilliert. Es wurden 149,8 g Produkt erhalten, das 84,1 % 2,4-Dimethyl-pyrrol enthielt. Das entsprach einer Ausbeute von 70 %, bezogen auf 2,4-Dimethyl-3 ,5-bis-methoxycarbonyl-pyrrol.

### Beispiel 2

420 g (2,0 mol) 2,4-Dimethyl-3,5-bis-methoxycarbonyl-pyrrol wurden in 1200 g 20 %iger Natronlauge suspendiert und die Mischung erhitzt. Der Feststoff ging dabei allmählich in Lösung. Bei 92°C setzte bereits eine heftige Gasentwicklung ein; dabei wurde ein Teil der Dicarbonsäure decarboxyliert. Das bei der Verseifung entstandene Methanol wurde kontinuierlich abdestilliert, bis nur noch hauptsächlich Wasser überging. Das verbliebene Gemisch wurde dann mit 250 g 50 %iger Schwefelsäure neutralisiert (pH etwa 7,5) und am Rückflußkühler mit Wasserabscheider zum Sieden erhitzt. Das 2,4-Dimethyl-pyrrol schied sich im Verlauf von wenigen Stunden als gelbe obere Phase ab. Die wäßrige Phase wurde kontinuierlich in das siedende Gemisch zurückgeleitet. Insgesamt wurde 152,5 g organische Phase erhalten. Diese enthielt 8,1 % Wasser, der Rest bestand zu 99,1 % aus 2,4-Dimethyl-pyrrol, ermittelt als Flächenprozente durch Gaschromatographie mittels Flammenionisationsdetektor. Das entsprach 138,9 g 2,4-Dimethyl-pyrrol (73 % d. Th.).

## Patentansprüche

1. Verfahren zum Herstellung von 2,4-Dimethyl-pyrrol, bei dem ein 2,4-Dimethyl-3,5-bis-alkoxycarbonyl-pyrrol mit starkem Alkali zur Dicarbonsäure verseift und die Dicarbonsäure thermisch decarboxyliert wird, dadurch gekennzeichnet, daß man den Dicarbonsäurealkylester mit 10 bis 30 %iger wäßriger Alkalilauge so lange unter Rückfluß erhitzt, bis der Feststoff in Lösung gegangen ist, das Reaktionsgemisch mit Säure neutralisiert und weiter am Rückfluß bis zur vollständigen Decarboxylierung erhitzt.

2. Verfahren nach Anspruch 1, dadurch gekennzeichnet, daß man als Alkalilauge Natronlauge einsetzt.

3. Verfahren nach Anspruch 1, dadurch gekennzeichnet, daß man die Lauge mit einer starken nichtflüchtigen Mineralsäure neutralisiert.

4. Verfahren nach Anspruch 1, dadurch gekennzeichnet, daß man ein 2,4-Dimethyl-3,5-alkoxycarbonyl-pyrrol mit 1 bis 4 C-Atomen in der Alkoxygruppe einsetzt.

5. Verfahren nach Anspruch 1, dadurch gekennzeichnet, daß man den Dicarbonsäurealkylester 5 bis 15 Stunden zur Verseifüng mit Alkalilauge am Rückfluß erhitzt.

6. Verfahren nach Anspruch 1, dadurch gekennzeichnet, daß man das Verseifungsprodukt zur Decarboxylierung unter Druck auf 130 bos 145°C am Rückfluß erhitzt.

7. Verfahren nach Anspruch 1, dadurch gekennzeichnet, daß man das Verseifüngsprodukt zur Decarboxylierung unter Normaldruck am Rückfluß erhitzt und das mit dem Wasserdampf übergehende 2,4- Dimethyl-pyrrol kontinuierlich aus dem Gleichgewicht abtrennt.

8. Verfahren nach Anspruch 1, dadurch gekennzeichnet, daß man zur Verseifung einen Überschuß an starkem Alkali einsetzt.
